# EUROPEAN PATENT APPLICATION

(11) **EP 4 643 833 A1**
(43) Date of publication of application: **05.11.2025**
(21) Application number: 24173798.0
(22) Date of filing: 02.05.2024
(51) Int. Cl.: A61F 13/42, A61B 5/08, G01N 27/00

(54) **SENSOR-INTEGRATED GARMENT AND ASSEMBLY THEREOF**

(71) Applicant: Abena Holding A/S, 6200 Aabenraa (DK); MediSens Wireless, Inc., Santa Clara, California 95051 (US)
(72) Inventor: Raut, Nitin, Sunnyvale, 94086 (US)
(74) Representative: Patrade A/S

(57) **Abstract**

The invention relates to assemblies and a network system, wherein a sensor array (11; 16; 17) operably incorporated into a disposable garment (10) and configured for attachment to a reusable transmitter (30). Together the garment and transmitter assembly create an integrated unit assembly allowing the garment to collect store and transmitter data to a central monitoring facility.

The garment (10) has specialized features that reduce the cost and complexity of manufacturing and provide an ease of use for healthcare workers using the integrated assembly to enable monitoring of individual patients.

## Description

### FIELD OF THE INVENTION

The invention relates to assemblies and a network system, wherein a sensor array operably incorporated into a disposable garment and configured for attachment to a reusable transmitter. Together the garment and transmitter assembly create an integrated unit assembly allowing the garment to collect store and transmitter data to a central monitoring facility.

The garment has specialized features that reduce the cost and complexity of manufacturing and provide an ease of use for healthcare workers using the integrated assembly to enable monitoring of individual patients.

### BACKGROUND OF THE INVENTION

The development of inexpensive medical sensors has allowed sensing functions to be incorporated into many different types of garments. Printed ink sensors that are printed with functional inks are particularly useful because the sensor elements can be implemented on material layer or layers during manufacture of the garment and the assembly of individual elements of the sensor can form a sensor array(s) that allows a wide range of detection functions to be available for patient monitoring of a range of healthcare data inputs that may be individualized to a particular patient by correlating the sensor signal to the garment used with an individual patient under care. In particular, the use of sensors in garments to advise health care workers of the status or condition of a patient is valuable to provide updated information of patient status while preserving healthcare provider resources. In these garments, sensors are incorporated into a fabric or synthetic layer of material that are formed together to create the garment. In the newest designs, the sensors can be paired with an electronic transmitter that gathers data from the sensor array and transmits information to a receiver to receive the data.

Because these items are designed to be wearable, the sensor has to be incorporated into a soft and flexible material that can be worn by a patient and the garment must closely conform to the shape of the body and be both comfortable and functional. Also, many garments such as diapers, pads, some bedding items, and others are designed to be disposable and may require multiple changes per day. To be useable in such a patient setting, the garment must be designed to be efficiently manufactured using minimal materials and components that are cost effective and susceptible to mass production.

Where a wearable sensor array is incorporated into a garment, the garment is advantageously connected to a central patient monitoring system monitored by doctors, nurses, caregivers, or other medical personnel. A transmitter is operably connected to the sensor incorporated into the garment and certain data is collected from the sensor array and transmitted to the central patient monitoring system. The data can include a wide variety of parameters and information including the type of garment being worn by the patient, the sensing parameters of the individual garment worn by the patient. This particularized sensing data may be based on a model or type of garment created by the manufacturer or may be specific enough to identify the particular unit of manufacture or electrical characteristics of each particular garment.

The data also preferably include an indication that the sensor is operating correctly and that the transmitter is correctly transmitting sensed data to the central monitoring facility. When disposable garments are used, the transmitter portion may be reusable so that the garment can be discarded after use and the sensing unit reused with a replacement garment. Typically, the transmitter is designed for releasable attachment at a point along the sensor array where the transmitter is attached to an electrically connected portion of the sensor array. In this configuration, the transmitter is clipped onto a portion of the garment containing an element of the sensor array that permits the transmitter to gather and transmit data from the entirety of the array incorporated into the garment.

Making an electrical connection between a removable transmitter and a printed conductive ink sensor and other printed features on soft substrates is a challenging task. The transmitter has to be easily clipped onto the garment and also be easily removable when the garment is discarded. Also, to reliable collect data from the sensor array, the transmitter must faithfully make an electrical connection at the point of attachment to the entirety of the array conductive elements of the array to the ability to gather data will be lost. Thus, both the garment and the transmitter typically incorporate mating features or other structural modifications that serve to establish and maintain a physical and electrical connection between the garment and the transmitter at the attachment point where the transmitter is clipped onto the garment.

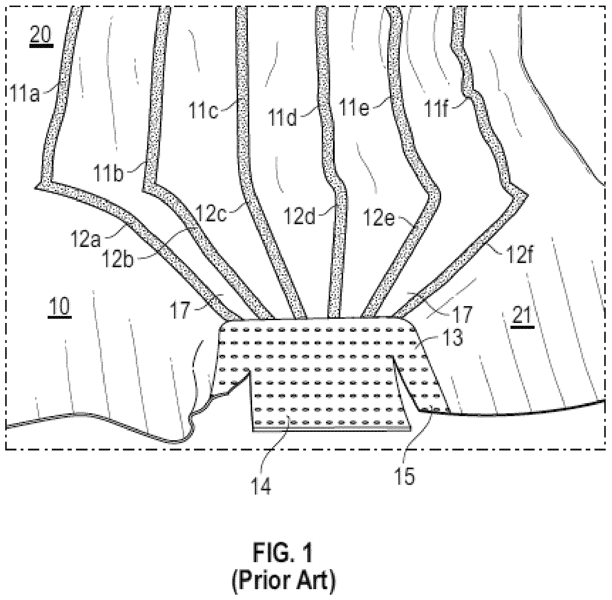

United States Patent Publication 2003001147 at Figure 3 describes the coupling apertures and prongs to secure a diaper having a sensor unit to a transmitter unit and uses contact/connecting rings in one of two panels in the transmitter to engage and secure the garment to the transmitter to maintain a secure electrical connection. This design requires significant modification to each of the structure of each diaper and the transmitter so that a secure physical and electrical connection is established and maintained during the sensing period once the diaper is worn by a patient.
Another option to create a physical and an electrical connection is achieved by adding an additional structure formed from a stiffening material to the contact area (on a non-electrically conductive side) of a defined region of the garment where the transmitter is attached to provide a defined area where the transmitter must be clipped to the sensor array (See Figure 1 below). The necessary manufacturing process that adds the stiffening material adds a discrete manufacturing step and corresponding complexity and cost to the manufacturing process. Many large production volume products, and diapers in particular, are produced in a process where large rolls of preformed material are cut into individual units as the material is passed through the manufacturing, assembly, and packaging process.

Adding structural stiffening materials as a discrete layer such as a tab to diapers at the precise location of transmitter attachment in a fast-running diaper production line requires identifying the location where the tab is to be attached for each unit and requires a far more complex manufacturing step for the underlying materials or a separate step to add the structural material tabs to each unit. This requirement adds to the complexity and cost of manufacturing. While speed and efficiency of manufacturing favor removing any special structures incorporated into the garment to enable attachment of the transmitter unit, the removal will create practical challenges in the attachment of the transmitter unit by the healthcare worker or other user.

For example, the absence of a defined area with stiffening material will require the user to attach the transmitter to the garment at a region that is flexible and, in some cases consisting only of as few as two layers disposed at the margin of the garment. In the case of incontinence products, the layers could be as few as one substrate later containing the sensor array and absorbent materials and one comfort layer facing the patient. Stated simply, the approaches that allow ease and efficiency of manufacture tends to also yield a garment structure that makes the action of attaching the transmitter more difficult and there is a conflict or tradeoff between reaching these two important goals.

Thus, a need exists to design an improved design for a garment containing an electrical sensor array, including particularly in improved design for the portion of the garment that engages a removable and reusable transmitter device that is releasably attached to the garment. The reusable transmitter device that is clipped onto the garment to electrically connect the sensor array must also have design features that are specially engineered to accommodate the revised structure for the portion of the garment that engages the transmitter. The assembly of the garment in the transmitter must be simple and reliable to use for the daily healthcare providers of a target patient population that is greatly in need of innovation to reduce costs and improve healthcare outcomes. If an improved system could be developed, the system would encourage more widespread adoption of advanced garment-based sensor technologies that have been shown to preserve costs and improve patient outcomes.

### OBJECT OF THE INVENTION

It is an object of the present invention to provide an alternative to the prior art.

In particular, it may be seen as an object of the present invention to provide an assembly and system that solves the above-mentioned problems.

### DESCRIPTION OF THE INVENTION

The invention includes a sensor array operably incorporated into a disposable garment and configured for attachment to a reusable transmitter. Together the garment and transmitter assembly create an integrated unit assembly allowing the garment to collect store and transmitter data to a central monitoring facility. The garment has specialized design features that reduce the cost and complexity of manufacturing and provide an ease of use for healthcare workers using the integrated assembly to enable monitoring of individual patients. Features of both the garment and the transmitter ensure that the connection created by the healthcare provider when making the assembly of the garment of the assembly are quick and accurate and provide a confirmation that the garment and the transmitter have been correctly combined to enable the data collection functions.

The invention also includes methods of manufacturing diapers to achieve the advantages describes above. One aspect of the invention is that advantageous manufacturing methods require the removal of existing structural features of the garment-transmitter combination that are ordinarily specifically included in the garment design to enable ease of the garment connector connection. Thus, an aspect of the invention includes design features of the transmitter and features of the garment where other structures are expressly removed for ease of manufacture and the disadvantages or removing these structures are overcome by the invention of other design features that overcome the removal of these structural features.

Specifically with respect to the garment, the invention includes the absence of any structure that provides rigidity or stiffness to the portion of the diaper where the transmitter is attached. This portion is defined as that portion at the outer edge or margin of the product where the transmitter is attached to create an electrical connection to the sensor array. The target area may also be referred to as a landing pad because the transmitter must be attached within a defined area and the attachment point of the garment, typically along the edge, for sensing and transmitting functionality to be established. Within this portion, a portion of the sensor array is disposed and arranged such that electrical connections formed by attachment of the transmitter at this portion form a complete electrical connection to the array. In a preferred embodiment the sensor array is narrowed to converge within this portion into a compressed pattern that match electrodes contained within the transmitter.

In one embodiment, the sensor array within the garment is comprised of substantially parallel conducting lines running the length of the garment wherein the individual sensor array lines deflect at an intermediate point between the absorbent portion of the garment and the attachment portion of the transmitter such that overall width of the sensor array narrows from the absorbent portion towards the edge of the garment. In a particularly preferred embodiment, the sensor array within the transmitter attachment portion is also a series of substantially parallel lines sized to engage a series of parallel conductors disposed within a portion of the transmitter designed to receive the fabric or substrate layers comprising the garment and containing the sensor array. As described below, one preferred design for the transmitter housing contains a visibly transparent portion that allows the healthcare worker attaching the transmitter portion to the garment to ensure proper connection between the transmitter and the garment by visual guides incorporated into the garment, the transmitter transparent portion or both.

In addition to the absence of stiffening substrates or other materials providing rigidity at the point of attachment of the transmitter, the selection and construction of the materials at the transmitter attachment portion are specially shaped, oriented, and organized as layers to facilitate attachment of the transmitter without added stiffening structures. This capability requires a modification to the coupling features of the transmitter, optimized features in the garment adage where the portions of the sensor array are available for coupling to the transmitter, and a workable assembly between the garment and the transmitter such that the transmitter can be easily attached, verified to be operational, and used to begin a sensing session once the garment is attached to a patient.

The garment product connection point is an unstiffened thin substrate designed to precisely align a selected region of the sensor array with the electrical connectors of the transmitter. The Clip/transmitter is specifically configured to align the electrodes of the sensor read/measurement circuit to the sensor assembly at the edge of the garment. The transmitter is comprised of a housing that has integrated structures to engage the sensor portion of the garment and to establish a secure electrical connection without modification of the transmitter attachment portion to add rigidity. The transmitter housing has a rotatable portion that rotates around an axis that is substantially parallel to the garment edge. The transmitter housing may also have a transparent portion shaped and oriented to allow a user to align the electrical connectors on the transmitter to a mating orientation of elements of the sensor array.

As noted above, the assembly of the invention enables electrical contact without adding any stiffening element or structure and also provides secure attachment of the transmitter to the garment. The transmitter housing contains attachment, locking, and biasing elements that force a portion of the sensor array into fixed physical contact and conductive electrical contact with electrical connectors on the transmitter. The portion of the sensor array disposed within the transmitter attachment region may have visual guides that are viewable through the transparent portion of the transmitter housing and assist the user in proper attachment of the transmitter so that a sensing session can begin. Rotation of the locking element around the axis formed from a hinge in the transmitter housing both attaches the transmitter to the garment at the designated region and also establishes the electrical connector.

Typically, there is a comfort layer, which serves as the layer making contact with the skin. Incontinence products move the fluid and/or other insults away from the skin and into the absorbent core. This comfort layer needs to be separated to provide a robust electrical contact. In order to attach the Clip to the printed sensor, the landing pad area is left without a glue creating a virtual pocket, which allows the layers (which are normally glued or sealed) to remain separate. This lack of glue also allows the landing pad area to retain electrical characteristics needed to make a robust electrical contact. A direct contact is needed in such an embodiment to make the electrical contact.

This layer, however, is quite thin and separating the comfort layer from the backsheet (where the sensor is printed) can become tedious and frustrating. We propose structures that will overcome this need and allow us to make an electrical contact with the printed landing pad through the comfort layer. In one embodiment the structures are built on the electrical contacts directly which allows them to penetrate the thin comfort layer and make contact with the printed landing pads.

In other embodiments, these structures are built on the clamping pads and force the printed substrate to make contacts by distending the printed surface and making a contact through the comfort layer onto the landing pad on the Clip/transmitter. We can also use structures on both clamping pads and Clip landing pads at the same time.

In addition to the rotatable locking feature and housing structure, the housing of the clip-on transmitter also contains clamping structures to cause contact to be created between the conductive ink printed sensor array and the electrical connectors contained within the transmitter unit. The transmitter unit will also contain circuitry for wireless transmission of array data gathered from the sensor array during a sensing session. The clamping structures engage a portion of the substrate, typically a substrate having the printed sensor array incorporated therein although the substrate may take a variety of forms including mere filler material or complex multi-layer structures having functional layers in discrete arrangements. The clamping function of the transmitter housing creates a stable electrical connection between the sensor array and the electrical connectors housed in the transmitter clip. In some embodiments, the clamping features contain one or more edges that penetrate a layer of the garment at the transmitter attachment point. Typically, cooperating structures are oriented between the sensor array and the electrodes of the transmitter unit to create form an electrical connection between one portion of the sensor array at the transmitter attachment region and one electrical connector within the transmitter. Visual or other feedback confirms to the user that all available connections have been made. In other embodiments the clamping layer may be made soft so that the tolerances on the connection points and assembly can be increased, leading to lower cost of manufacturing.

The transmitter has an internal circuit and identity database to confirm that the attachment is correct and may have a light or other visual element to confirm that the transmitter is properly attached to the underlying garment such that the electrical connectors are forming a competent electrical connection with the sensor array. The transmitter may also have a split or groove formed in the housing allowing a portion of the garment comprising the portion of the sensor disposed within the transmitter attachment region to be contained within this portion of the transmitter housing. Rotation of the locking mechanism secures the transmitter to the garment and, as above, creates an electrical connection between the sensor array and the transmitter.

It should be noted that all features, elements, components, functions, and steps described with respect to any embodiment provided herein are intended to be freely combinable and substitutable with those from any other embodiment. If a certain feature, element, component, function, or step is described with respect to only one embodiment, then it should be understood that that feature, element, component, function, or step can be used with every other embodiment described herein unless explicitly stated otherwise. This paragraph therefore serves as antecedent basis and written support for the introduction of claims, at any time, that combine features, elements, components, functions, and steps from different embodiments, or that substitute features, elements, components, functions, and steps from one embodiment with those of another, even if the following description does not explicitly state, in a particular instance, that such combinations or substitutions are possible. It is explicitly acknowledged that express recitation of every possible combination and substitution is overly burdensome, especially given that the permissibility of each and every such combination and substitution will be readily recognized by those of ordinary skill in the art.

While the embodiments are susceptible to various modifications and alternative forms, specific examples thereof have been shown in the drawings and are herein described in detail. It should be understood, however, that these embodiments are not to be limited to the particular form disclosed, but to the contrary, these embodiments are to cover all modifications, equivalents, and alternatives falling within the spirit of the disclosure. Furthermore, any features, functions, steps, or elements of the embodiments may be recited in or added to the claims, as well as negative limitations that define the inventive scope of the claims by features, functions, steps, or elements that are not within that scope.

The above described object and several other objects are intended to be obtained in a first, second and/or third aspect of the invention.

In some first aspects, the techniques described herein relate to an assembly for monitoring incontinence, including:
In some aspects, the techniques described herein relate to a disposable garment having an absorbent layer, located between a first marginal edge and a second marginal edge of the garment, wherein the first and second margin edges have a flexibility and rigidity defined by the material layers forming the marginal ends.

In some aspects, the techniques described herein relate to an external backing layer in fluid communication with the absorbent layer, and a contact layer disposed on an inner surface of the external backing layer between the absorbent layer and the external backing layer, and extending between the first and second marginal ends of the garment.

In some aspects, the techniques described herein relate to wherein the second marginal end is included of an intact linear segment at the terminal end thereof that is sized and oriented to receive a transmitter unit. This embodiment may be referred to as tables due to the absence of a separate support structure separate from the layers of the garment and where the portion for attachment of the transmitter unit has an equivalent flexibility and rigidity as the first marginal end and such that an absence of additional material adding flexibility or rigidity and wherein the intact linear segment has a width with boundaries containing terminal ends of a series of paired conducting lines of a sensor array.

In some aspects, the techniques described herein relate to an assembly, wherein the sensor array having a single unique electronic signature included, further including a reusable transmitter unit having an aperture to receive the linear segment of the second marginal edge containing the terminal end of the sensor array, means to engage the terminal ends of the series of paired conducting lines at the intact linear segment of the second marginal edge of the garment, a fixed array of electrodes oriented to engage the terminal ends of the series of paired conducting lines such that engagement means causes an individual electrode of the transmitter unit to electrically connect a single conducting line of the sensor array at the intact linear segment of the second marginal edge of the garment.

In some aspects, the techniques described herein relate to an assembly, wherein a plurality of the lines in the sensor array have an angled portion disposed between a portion of the line proximate to the absorbent material and the terminal end located at the second marginal end of the garment.

In some aspects, the techniques described herein relate to an assembly, wherein the terminal ends of the series of paired conducting lines are a set of parallel lines contained within the width of the intact linear segment and terminating at the terminal end of the second marginal end.

In some aspects, the techniques described herein relate to an assembly, wherein the transmitter unit has an open position and a closed position, wherein the open position receives the intact linear segment and the closed position establishes an electrical connection between the sensor array and the transmitter unit.

In some aspects, the techniques described herein relate to an assembly, wherein the means to engage the terminal ends of the series of paired conducting lines at the intact linear segment of the second marginal edge of the garment includes a rotatable element and a latch mechanism to maintain the electrical connection when the transmitter unit is in the closed position.

In some aspects, the techniques described herein relate to an assembly, wherein the rotatable portion of the transmitter assembly is further included of means to force engagement between the terminal ends of the series of paired conducting lines of the sensor array and the individual electrodes of the transmitter unit.
In some aspects, the techniques described herein relate to an assembly, wherein the transmitter unit has circuitry to determine if the electrical connection has been made and an indicator to confirm the electrical connection to a user.

In some aspects, the techniques described herein relate to an assembly wherein a portion of the transmitter unit is transparent and the terminal ends of the series of paired conducting lines of the sensor array are visible through the transparent portion and in the aperture of the transmitter unit.

In some second aspects, the techniques described herein relate to an assembly to monitor incontinence in a patient including:
In some aspects, the techniques described herein relate to a disposable garment having an absorbent layer and formed from a fluid impermeable outer layer having a sensor array disposed on an inner surface thereof and in fluid communication with the absorbent layer and a non-woven comfort layer covering the absorbent layer and disposed between the sensor array and the absorbent layer and the patient,-wherein the impermeable outer layer and the comfort layer have a marginal edge having a terminal end with a flexibility and rigidity defined by the material layers forming the marginal end, --wherein the terminal end is intact across a transmitter-unit connecting area consisting essentially of marginal edges of the fluid impermeable layer and the comfort layer in the absence of additional material adding flexibility or rigidity.

In some aspects, the techniques described herein relate to the sensor array included of a series of conducting lines formed by conducting ink --parallel and of substantially equal width] layered on the inner surface of the external backing layer and in fluid communication with the absorbent material to form linear sensing regions for detecting a gradient of non-binary conductance values between the conducting lines and-wherein the conducting lines are continuous and each terminate in the transmitter-unit connecting area in the absence of additional material adding flexibility or rigidity and in a configuration matching a fixed array of electrodes in a reusable transfer unit.

In some aspects, the techniques described herein relate to the reusable transmitter unit sized and shaped to engage the transmitter-unit connecting area and having an open position and a closed position and wherein a housing of the transmitter unit contains a fixed array of electrodes and means to force the terminal ends of the series of conducting lines at the intact linear segment of the marginal edge of the garment into an electrical connection with the fixed array of electrodes, --wherein the forcing means creates a multi- layered combination in the closed position including an individual electrode of the transmitter unit in electrical connection with a single conducting line of the sensor array at the intact linear segment, and the a forcing means exerting to create the electrical connection.

In some aspects, the techniques described herein relate to an assembly, wherein the transmitter unit has a groove traversing a lateral surface of the transmitter, wherein the groove has a depth to receive the marginal edge of the garment and a width to receive the sensor array in the transmitter-unit connecting area of the garment.

In some aspects, the techniques described herein relate to an assembly, wherein the transmitter unit is further included of a series of projections aligned across the forcing means such that in the closed position the projections create the electrical connection by bringing a member of the series of paired conducting lines into electrical contact with a member of the fixed array of electrodes.

In some aspects, the techniques described herein relate to an assembly, wherein the projections penetrate the comfort layer proximate to the member of fixed array of electrodes.

In some aspects, the techniques described herein relate to an assembly, wherein the terminal ends of the pairs of conducting lines are an array of parallel lines in the transmitter-unit connecting area spaced apart to match the distance between members of the fixed array of electrodes in the transmitter unit.

In some aspects, the techniques described herein relate to an assembly, wherein the terminal ends of the pairs of conducting lines are bounded by a visual marker for aligning the terminal ends with an aperture in the housing of the transmitter unit that receives the marginal edge of the garment.

In some aspects, the techniques described herein relate to an assembly further including a computer network receiving wireless information from the transmitter unit that identifies a single and unique electronic signature for each single garment.

In some third aspects, the techniques described herein relate to a network system for detecting incontinent events in an individual patient including: (a) a garment having a sensor array in fluid communication with absorbent material disposed in the garment, wherein the garment has a marginal edge with a flexibility and rigidity defined by the material layers forming the marginal end layer in the absence of additional material adding flexibility or rigidity to an intact attachment region located at the marginal edge of the garment; (b) a sensor array having an electrical signature that may be unique to each garment and may include an identifier as part of the electrical signature to identify the particular garments, configuration of garment, or protocol to be used in analyzing data from the sensor array, and including conducting lines having terminal ends thereof aligned in the intact attachment region; (c) a transmitter unit having an aperture sized and shaped to receive the intact attachment region, a fixed array of electrodes oriented to match the alignment of the terminal ends of the conducting lines, means for releasably securing the marginal edge to the transmitter unit, and an indicator confirming an electrical connection between the electrodes of the transmitter unit and the terminal ends of the sensor lines and a connection to a wireless network; and (d) a data processing unit receiving data over the wireless network that correlates the presence of fluid in the absorbent material and detected by the sensor --with the unique electronic signal of each garment, and optionally --a user interface to identify the individual patient with an incontinent event.

In some aspects, the techniques described herein relate to a network system, wherein the network system monitors a plurality of patients

In some aspects, the techniques described herein relate to --and the data processing unit distinguished between individual patients based on information received from the sensor array.

Other systems, devices, methods, features and advantages of the subject matter described herein will be or will become apparent to one with skill in the art upon examination of the following figures and detailed description. It is intended that all such additional systems, devices, methods, features and advantages be included within this description, be within the scope of the subject matter described herein, and be protected by the accompanying claims. In no way should the features of the example embodiments be construed as limiting the appended claims, absent express recitation of those features in the claims.

The first, second and third aspect of the present invention may each be combined with any of the other aspects. These and other aspects of the invention will be apparent from and elucidated with reference to the embodiments described hereinafter.

Embodiments from the system/product should be seen as applicable to the method, and embodiments from the method should be seen as applicable for the product/system.

### DESCRIPTION OF THE DRAWING

Various examples are described hereinafter with reference to the figures. Like reference numerals refer to like elements throughout. Like elements will, thus, not be described in detail with respect to the description of each figure. It should also be noted that the figures are only intended to facilitate the description of the examples. They are not intended as an exhaustive description of the claimed invention or as a limitation on the scope of the claimed invention. In addition, an illustrated example need not have all the aspects or advantages shown. An aspect or an advantage described in conjunction with a particular example is not necessarily limited to that example and can be practiced in any other examples even if not so illustrated, or if not so explicitly described.

Exemplary embodiments of the invention are described in the figures, whereon:
Fig. 1 is an exemplary prior art approach to provide an attachment point for an electrical apparatus for gathering data from an electrical sensor array incorporated into a garment.
   Referring to Figure 1 showing an example of a disposable incontinence product known in the art, the underlying structure of the garment 10 is at least two layers comprising an absorbent substrate 20 (see also figures 2 and 3 below) and a non-woven comfort layer 21 that contacts the skin of the patient and separates the skin from the absorbent material incorporated into the absorbent substrate 20 of the garment 10.

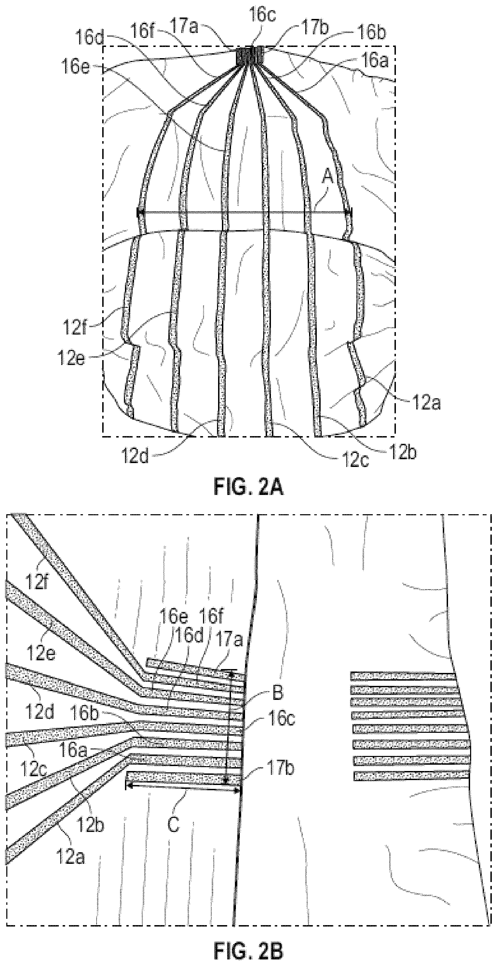

Fig 2A is a view of a sensor array disposed within a garment showing the region containing substantially parallel-non-interconnecting lines running a length of the garment to provide liquid-sensing and other functionalities by sensing a gradient of conductivity along the sensor array and between pairs of individual lines.
Fig. 2B shows the arrangement of the individual lines of the sensor array at an edge of the garment and one configuration for layers comprising the garment itself and an arrangement for selectively exposing the terminal ends of the sensor array for attachment by the transmitter unit.

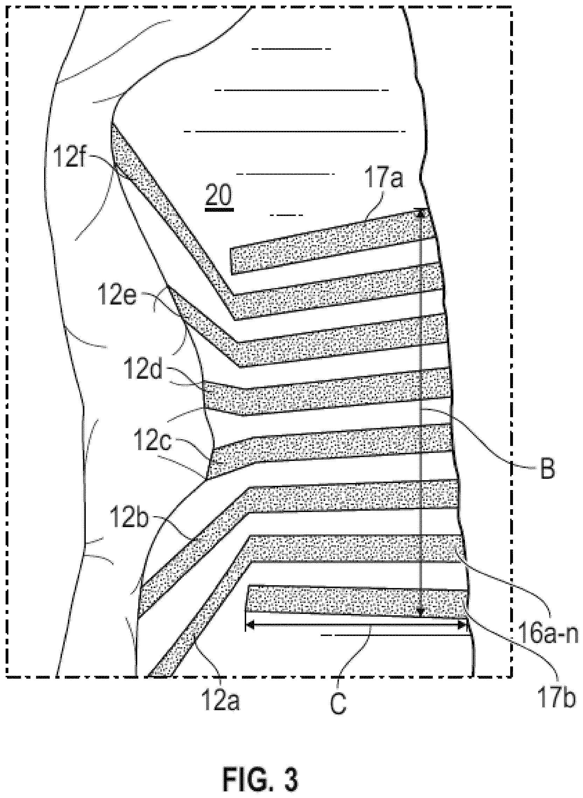

Fig. 3 is a dedicated region of the garment containing the portions of the array in the sensor attachment region and aligned to be engaged by the electrical connectors of the transmitter unit and that may be separated from the absorbent substrate layer at the transmitter binding region.

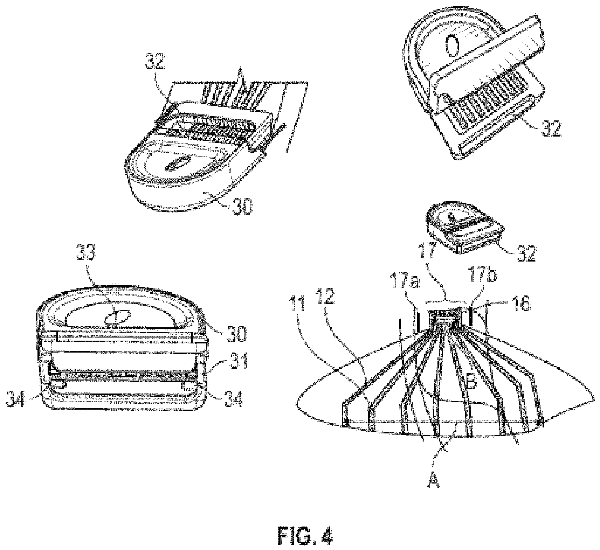

Figure 4 shows a transmitter unit having an aperture for receiving the sensor assembly at the transmitter attachment portion of the marginal edge of the garment.

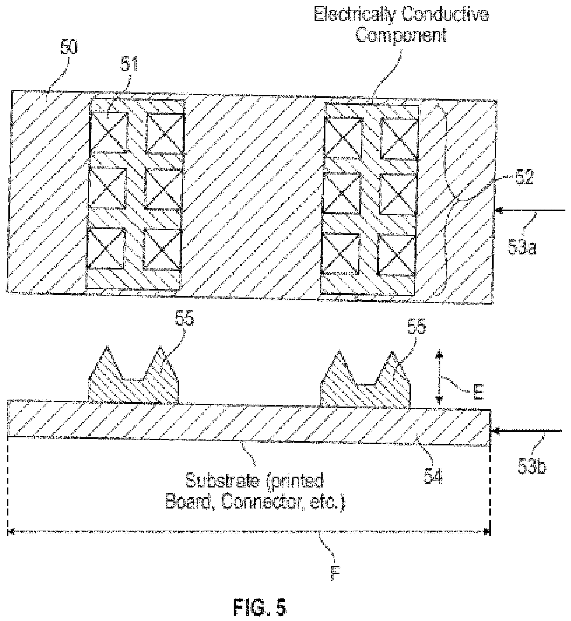

Fig. 5 contains bottom and front views of the electrical connector contained in the transmitter element showing the clamping features that penetrate the inner layer of the garment.

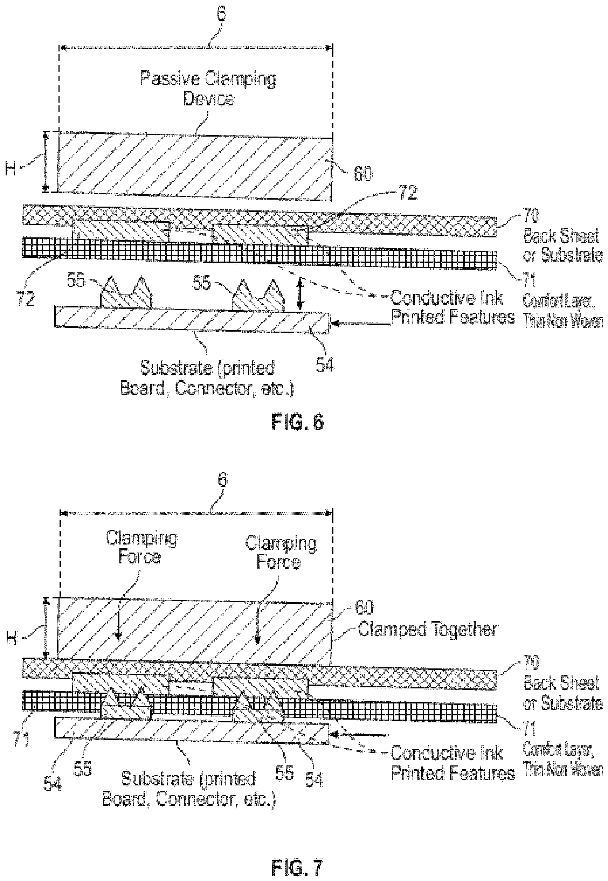

Fig. 6 is a cross-sectional view showing a cable clamping feature of the transmitter housing position to cause the sensing elements of the sensor array to be biased into position to create the electrical connection between the sensor array and the transmitter.
Fig. 7 shows the clamping force (at the downward arrows) creating the electrical connection between the array and the electrical connectors of the transmitter.

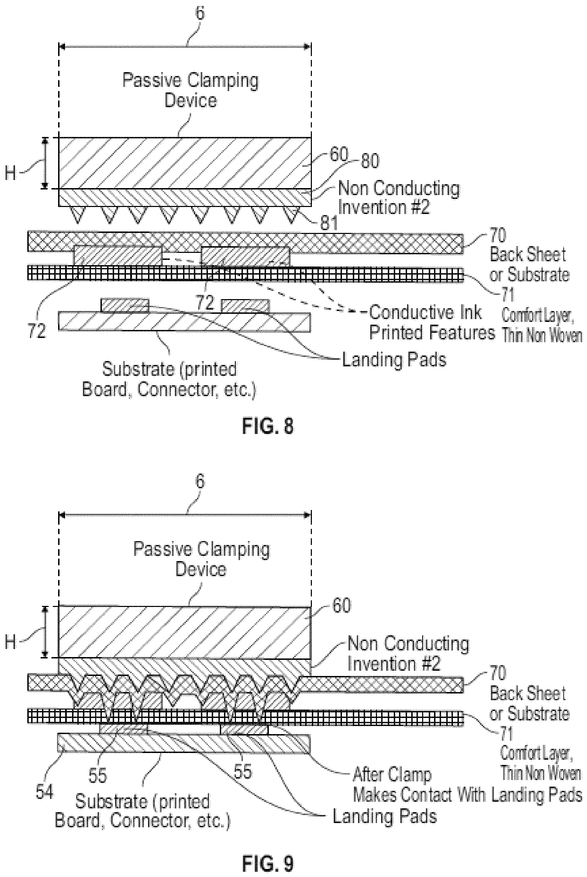

Fig 8 is a cross-sectional view of a clamping mechanism incorporating a non-conducting layer into the clamping mechanism to penetrate the absorbent substrate containing the sensor array to bias the sensor array lines into electrically conductive contact with the electrical connectors of the transmitter.
Figure 9 Example showing the product for connection.

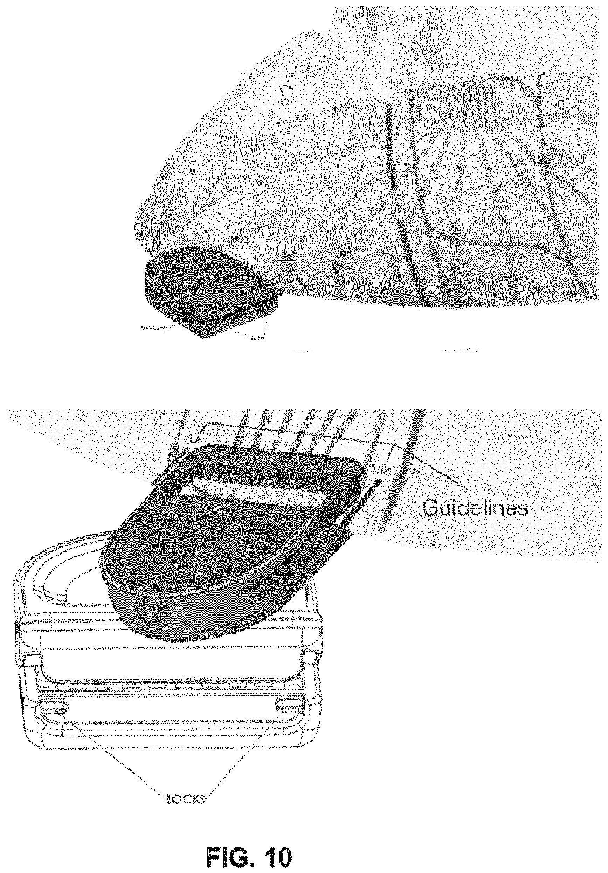

Fig 10 illustrates a visual alignment feature of the present invention wherein discrete markers proximate to the outer edges of the sensor array at the landing pad area direct the user to attach the transmitter unit between the visual alignment guides to align individual electrodes in the transmitter unit to the sensing lines of the array.

### DETAILED DESCRIPTION OF THE INVENTION

Exemplary examples will now be described more fully hereinafter with reference to the accompanying drawings. In this regard, the present examples may have different forms and should not be construed as being limited to the descriptions set forth herein. Accordingly, the examples are merely described below, by referring to the figures, to explain aspects.

Throughout the specification, when an element is referred to as being "connected" to another element, the element is "directly connected" to the other element, "electrically connected", "fluidic connected" or "communicatively connected" to the other element with one or more intervening elements interposed there between.

The terminology used herein is for the purpose of describing particular examples only and is not intended to be limiting. As used herein, the terms "comprises" "comprising" "includes" and/or "including" when used in this specification specify the presence of stated features, integers, steps, operations, elements, and/or components, but do not preclude the presence or addition of one or more other features, integers, steps, operations, elements, components, and/or groups thereof.

Unless otherwise defined, all terms used herein (including technical and scientific terms) have the same meaning as commonly understood by those skilled in the art to which this invention pertains. It will be further understood that terms, such as those defined in commonly used dictionaries, should be interpreted as having a meaning that is consistent with their meaning in the context of the relevant art and will not be interpreted in an idealized or overly formal sense unless expressly so defined in the present specification.

Before the present subject matter is described in detail, it is to be understood that this disclosure is not limited to the particular embodiments described, as such may, of course, vary. It is also to be understood that the terminology used herein is for the purpose of describing particular embodiments only and is not intended to be limiting. In the above figures and the following discussion, an absorbent diaper is used to demonstrate the features and elements of the invention. However, one of ordinary skill in the art will readily appreciate that the structures, methods, and elements of the invention could be applied to any garment containing a sensor array and having the need to attach a transmitter element to a soft and flexible substrate where precise alignment of electrical connectors in the transmitter to elements of the sensor array is critical to gathering sensor data.

A disposable diaper, typically, consists of an absorbent pad sandwiched between two sheets of nonwoven fabric. The pad is specially designed to absorb and retain body fluids, and the nonwoven fabric gives the diaper a comfortable shape and helps prevent leakage.

Today's diapers are not only highly functional, but they also include advanced features such as special sizing and coloring for specific gender and age, color change indicators to , incorporated sensor arrays and reattachable Velcro-type closures

At this point in the process, the incontinence garment is assembled by integrating the printed sensor back sheet with rest of the components of the incontinence product. The assembled diaper may have other attachments, such as strips of tape or other adhesive elements to act as closures. The long roll is then cut into individual diapers. As shown in Figure 1, where a sensor array is disposed on the back sheet, additional structural formation is necessary to provide rigidity for the point of attachment of the transmitter unit. To provide this structure, each individual diaper must be separated from the continuous production, and a separate rigidity or support structure or "tab" must be individually and precisely attached with extremely tight tolerances at the portion of the edge of the diaper where the transmitter unit is to be attached. An otherwise continuous process, this step must be performed individually, or manually, and with a high degree of precision to ensure that the additional support structure conforms to the existing edge of the diaper and is aligned to permit attachment of the transmitter unit in such a way that communication with the transmitter unit and the sensor array will be achieved. This individual step detracts significantly from the economics and efficiency of incorporating a sensor into the diaper garment.

Moreover, when the added feature of the sensor array is important to the use of the diaper garment, any failure in quality control at step of individual attachment of the rigid support element renders the entire sensor array useless and defeats the functionality offered by the sensor array. Any significant variation in how the support structure is attached to the underlying diaper garment substrate will defeat the ability of the transmitter unit to be operably connected to the underlying sensor array.

Also, the traditional issues with quality control of the diaper garment's fit and comfort must be maintained even though the unit assembly is conformed for attachment of a transmitter unit along the edge of the garment to connect to the sensor array. Accordingly, particular attention must be paid to the configuration of the individual layers of the garment along the entire margin of the unit and the approximate edge where the individual elements of the sensor terminate. Because the edge of the garment where the transmitter unit attaches is critical to functionality, particular attention must be paid to the design, orientation, and size of the margin of the garment along the edge where the transmitter unit is attached and a configuration that avoids the necessity to attach an individual structural element for rigidity must be precise, reliable, and reproducible in a mass production assembly method. Therefore, to enable the functionality of the sensor array, the assembly, alignment, and orientation of each of the structural and sensing components must similarly be precise, reliable, and reproducible.

Referring to Figure 1 showing an example of a disposable incontinence product known in the art, the underlying structure of the garment 10 is at least two layers comprising an absorbent substrate 20 (see also figures 2 and 3 below) and a non-woven comfort layer 21 that contacts the skin of the patient and separates the skin from the absorbent material incorporated into the absorbent substrate 20 of the garment 10. The sensor array is comprised of individual printed ink conductive lines 11a-11f that run a substantial length of the garment to create the sensor array. The conductive lines 11a-11f deflect at an intermediate portion such that the individual printed sensor lines 12a-12f are disposed at an angle to the parallel lines of the sensor array 11a-11f. The angled-printed sensor lines 12a-12f terminate proximate to the transmitter attachment region 17 and converge in a tightly spaced parallel line configuration 16a-16f sized and configured to match the electrical connectors disposed in the housing of the transmitter. In this configuration, the sensor array is comprised of printed, electrically conductive spaced apart substantially parallel lines 11a-11f, and angled section of the sensor array 12a-12f that converge in terminate at the marginal end of the garment 10 to form a matching pattern of parallel lines disposed at the transmitter attachment region.

As noted above, it is deemed necessary to individually attached stiffening material or tab 13 to provide stiffness or rigidity at the point of attachment of the transmitter unit. In the absence of the tab 13, the material at the transmitter attachment region 17 is difficult to manually manipulate to attach the transmitter. The tab 13 may be separated into a dedicated central portion 14 having a longitudinal slit cut into the borders thereof that traverses both the absorbent substrate layer 20 and the nonwoven comfort layer 21 and that matches the dimensions of the outer portions of the transmitter housing. In this configuration, the user aligns the central portion 14 such that the lateral portions 15 lie outside the transmitter attachment region 17 and then manipulates the tab portion to come within the body of the housing of the transmitter for attachment. As noted above, although the incorporation of the tab 13 into the marginal edge of the garment 10 facet facilitates attachment of the transmitter, the positioning and attachment of the tab in the creation of the special receiving area for the transmitter requires a discrete manufacturing step that adds to the cost and complexity of the entire garment-sensor assembly.

Referring to Figures 2A, 2B, and 3, the invention provides a defined region for the transmitter attachment absent any additional stiffening material added to either the conductive or nonconductive portions of the sensor array 11a-11f that reach the marginal edge of the garment 10. This portion of the garment 10 has several structural and design features that enable attachment of the transmitter unit without the addition of the tab 13 at the attachment region 17. Absent the addition of the tab 13 the only structure between the absorbent substrate and the printed portion of the array 11a-11f terminating in the transmitter attachment region 17 comprising horizontal lines 16a-16f is the non-woven comfort layer 21. Unlike a configuration having a tab, the tabless transmitter attachment region 17 provides access to the sensor array if the non-woven layer is penetrated. Also, the entire transmitter region 17 across the lateral marginal edge of the garment 10 is intact without any longitudinal slits or other alterations to the structural integrity of either of the absent layer substrate 20 construction or penetration of the non-woven comfort layer 20 as manufactured. Figure 3 shows that in the tabless construction the both the conducting non conducting side of the transmitter attachment region 17 is readily accessed and certain design modifications to the transmitter as described below enable establishing a reliable electrical connection even in the absence of the tab.

Referring specifically to Figure 3, the tightly configured array of parallel lines contacting the marginal edge of the garment 10 are electrically accessible either by attaching electrodes to these portions of the sensor array directly or by penetrating the nonwoven comfort layer 21. Two ease the visual process of aligning the electrodes within the housing of the transmitter with these portions of the sensor array, lateral visual elements 17a, 17b may be formed in the substrate layer 20 so that each member of the sensor array 16a-16f is electrically connected with a single electrode in the transmitter housing. The use of visual elements 17a, 17b to align the sensor array to the transmitter housing is particularly preferred in the embodiment of the transmitter housing described below having a transparent portion such that the alignment of the visual elements 17a, 17b can be viewed through the transparent portion of the housing.

Further modifications to the transmitter housing to facilitate correct alignment and electrical connection of the sensor array to the transmitter electrodes are described below.

Referring to Figure 4, in various embodiments, a transmitter unit 30 having an aperture 31 for receiving the sensor assembly at the transmitter attachment portion 17 of the marginal edge of the garment 10. In one embodiment, the garment 10 includes visual guides 17a, 17b that are visible through a transparent portion 32 of the transmitter housing 30. The aperture 31 comprises locking elements 34 and a visual indicator 33 indicating that a competent electrical connection has been made between the sensor array and the transmitter clip 30. The aperture may assume any size and shape that conforms to the sensor assembly at the transmitter attachment portion 17. A rotatable hinge that captures a portion of the edge of the document near the transmitter attachment portion 17 May rotate about an axis generally parallel to the edge of the garment and has both an open position for receipt of the transmitter attachment portion 17 as well as a closed portion where the electrodes in the transmitter unit 30 are brought into alignment and a reversible locking configuration is achieved between the transmitter unit and the marginal edge of the garment 10. In one embodiment, the transmitter unit has a slot in the manner of a groove formed into the body of the unit for receiving the in the regions of the plurality of sensor lines. In that embodiment insertion of the leading edge of the garment into the slot or groove causes engagement of the transmitter unit electrodes with the corresponding regions of the sensor lines.

In the clip embodiment having a rotatable hinge or other element forming an open aperture, the clip itself may include a biasing structure that forces the terminal ends of the sensor lines in engagement with the electrical sensors disposed inside of the transmitter unit. Because the transmitter is engaging a portion of the garment that is comprised of multiple fabric or material layers, the action of causing the transmitter unit form a reversible locking engagement with the marginal edge of the garment involves a mechanical compression of the garment layers and preferably a perforation of one or more material layers to improve the electrical connection between the sensing lines in the transmitter unit.

As noted above, the structural modification to the garment 10 and the cooperation with the transmitter unit 30 as described above permit secure attachment of the transmitter clip unit 30 to the garment 10 while establishing the electrical connection for a sensing session. The following describes approaches of the invention to advantageously form the attachment without the use of a tab structure or various structural means for achieving the attachment function.

In the approach described in Figure 5, a geometric pattern is formed in one side of a substrate or an element of the transmitter unit 30, in this specific embodiment, an array of pyramid-shaped structures are arranged in a pattern to act as penetration structure 40 to connect the sensor array features on the absorbent substrate 20 to electronics (active or passive) integrated into the circuitry of the transmitter unit 30. The penetrating structure 40 can be a component on the printed circuit board, or any other substrate aligned in the housing of the transmitter 30 unit. The arrangement of flexible substrate with printed functional features (product), and the invention embodiment is shown in Figure 5. In a preferred embodiment, the perforating structures have a height E greater than the thickness of any material layers such as an absorbent substrate 20 or comfort layer 21 or combination thereof to ensure that the electrical connection is established and is secure. Once the entire arrangement is clamped together to make the connection, the invention features penetrate the comfort layer, either through the non-woven gaps, or by perforating the layer and makes a contact with the electrically conductive features on the substrate and a solid contact is established as in Figure 7. This embodiment can be manufactured in a variety of ways including stamped metal foils.

Referring to Figure 6, a passive clamping device 60 is oriented in alignment with the positioning of the conductive ink printed sensor lines and the sensor unit 30 substrate containing the matching electrodes. As explained in the earlier embodiment, the force exerted on the clamping device creates electrical connection by perforating the comfort layer in bringing the entire assembly into a locked, but releasable configuration for secure and stable electrical connection. Passive clamping device 60 may be comprised of a compressible material that is shaped and sized to conform to the width of the assembled sensor array at the sensing area at the edge of the garment. An advantage of this configuration is that the comfort layer does not have to be manipulated by hand to bring the sensor elements into contact with the individual electrodes. Referring to Figure 7, as assembled with applied clamping force to form the locked but releasable assembly, the penetrating structures 55 traverse the comfort layer 71 and bring the electrodes into electrically conductive contact with the printed conducting sensor lines.

This configuration is maintained until the clamping force is released and the transmitter unit 30 is reused with a fresh garment. Again referring to Figure 7, the sensor unit 30 viewed in cross-section would include top and bottom structures of the sensor unit 30 itself the edge of the garment disposed therebetween, sensor elements in contact with the entire range of sensor lines 11a-11f, wherein the width of the assembly is guided by visual alignment elements located on either side of alignment elements 17a, 17b and spanning the entire width B of the sensor lines oriented at the edge of the garment.

Referring to the embodiment of Figures 8 and 9, similar to the related configurations of Figures 6 and 7, Figures 8 and 9 illustrate an open position and a releasably locked position of an embodiment where the perforating structures 81 proximate to the passive clamping surface. In Figure 8, a set of one or more structures are disposed on a back sheet or substrate 70 and sized and oriented to penetrate the comfort layer 71. The perforating structures 81 should have an overall height adequate to traverse the entire width of the comfort layer 71 and may have a pointed configuration or simply extend linearly from the back sheet 72 traverse the comfort layer 71 and are made of nonconductive material to avoid interfering with the electrical signal coming from the sensor array 55 and being communicated electrodes disposed in the transmitting unit 30.

Referring to Figure 9, in the releasably locked configuration, an integrated assembly is formed to create a secure and stable electrical connection between the sensor array and the individual electrodes of the transmitter unit 30. Figure 9 shows the cross section after they are clamped together. In this embodiment 2 narrow zones distend the thin flexible substrate from the clamping side, and in turn distends the printed flexible features into a shape similar analogous to the electrical connection formed in Figure 7. This distention then cause the printed feature to break through the secondary layer and make contact with the electrical landing pad (typically on a printed circuit board).

Figure 10 illustrates a feature of the invention wherein visual alignment of the transmitter unit 30 are enabled by markings on the underlying garment located outside of the sensor array in the landing pad area such that proper placement of the sensor unit 30 between the visual alignment guidelines facilitates proper alignment of the electrodes to the underlying sensor lines and secure and stable electrical attachment of the transmitter unit 30 the sensor array.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

### REFERENCE LIST

10 Garment
11a-11f Ink conductive lines, sensor array
12a-12f Sensor lines, angled
13 Stiffening material
13 Tab
14 Central portion
15 Lateral portions
16a-16f Line configuration, configured to match the electrical connectors
17a-17b Visual guide / elements
17 Transmitter attachment region
20 Absorbent substrate
21 Non-woven comfort layer
30 Transmitter unit
30 Transmitter housing
30 Transmitter clip
31 Aperture
32 Transparent portion
33 Visual indicator
34 Locking elements
40 Penetration structure
60 Passive clamping device
71 Comfort layer
72 Back sheet
81 Perforating structures
E, Height (of perforating structures)
B, Width (of the sensor lines oriented at the edge of the garment)

## Claims

1. **An assembly** for monitoring incontinence, said system comprising:
- a disposable garment (10) having an absorbent layer (20):
- wherein said layer (20) is located between a first marginal edge and a second marginal edge of the garment (10), and
- preferably, wherein the first and second margin edges have a flexibility and rigidity defined by the material layers forming the marginal ends,
- an external backing layer in fluid communication with the absorbent layer (20), preferably extending between the first and second marginal ends of the garment (10),
- a contact layer disposed on an inner surface of the external backing layer between the absorbent layer (20) and the external backing layer.
wherein the second marginal end is comprised of an intact linear segment at the terminal end thereof that is sized and oriented to receive a transmitter unit (30), and preferably has an equivalent flexibility and rigidity as the first marginal end, and preferably wherein an absence of additional material is adding flexibility or rigidity, and
wherein the intact linear segment has a width with boundaries containing terminal ends of a series of paired conducting lines (11; 16; 17) of a sensor array.

2. An assembly according to claim 1, wherein the sensor array (11; 16) having an electronic signature comprised of the series of paired conducting lines (11; 16; 17) and formed by conducting ink.

3. An assembly according to one or more of the preceding claims, wherein
- the conducting lines (11; 16; 17) are substantially parallel and of substantially equal width printed on an inner surface of the external backing layer and in fluid communication with the absorbent material to form linear sensing regions between each pair of parallel lines for detecting a gradient of non-binary conductance values between the pairs of lines and along a majority portion of a length of the absorbent layer (20).

4. An assembly according to one or more of the preceding claims, wherein
- the conducting lines (11; 16; 17) are continuous and extend between a portion of the array proximate the first marginal edge and extend to the terminal ends of the series of paired conducting lines disposed in the terminal end of the second marginal edge.

5. An assembly according to one or more of the preceding claims, wherein
- the conducting lines (11; 16; 17) are spaced apart by a larger distance when in proximity to the absorbent material than when disposed in the terminal end of the second marginal edge.

6. An assembly according to one or more of the preceding claims, wherein the assembly further comprises:
a reusable transmitter unit (30) having an aperture (31) to receive the linear segment of the second marginal edge containing the terminal end of the sensor array (11; 16; 17).

7. An assembly according to one or more of the preceding claims, wherein the assembly further comprises:
means to engage the terminal ends of the series of paired conducting lines (11; 16; 17) at the intact linear segment of the second marginal edge of the garment (10)

8. An assembly according to one or more of the preceding claims, wherein the assembly further comprises:
- a fixed array of electrodes (55) oriented to engage the terminal ends of the series of paired conducting lines (11; 16; 17) such that engagement means causes an individual electrode (55) of the transmitter unit (30) to electrically connect a single conducting line of the sensor array (11; 16; 17) at the intact linear segment of the second marginal edge of the garment (10).

9. An assembly according to one or more of the preceding claims, wherein a plurality of the lines (11; 16; 17) in the sensor array have an angled portion (12) disposed between a portion of the line proximate to the absorbent material and the terminal end located at the second marginal end of the garment (10).

10. An assembly according to one or more of the preceding claims, wherein the terminal ends of the series of paired conducting lines (11; 16; 17) are a set of parallel lines contained within the width of the intact linear segment and terminating at the terminal end of the second marginal end.

11. An assembly according to one or more of the preceding claims, wherein the transmitter unit (30) has an open position and a closed position, wherein:
- the open position receives the intact linear segment (17), and
- the closed position establishes an electrical connection between the sensor array (11; 16; 17) and the transmitter unit (30).

12. An assembly according to claim 7, wherein the means to engage the terminal ends of the series of paired conducting lines (11; 16; 17) at the intact linear segment of the second marginal edge of the garment comprises a rotatable element and a latch mechanism to maintain the electrical connection when the transmitter unit (30) is in the closed position.

13. An assembly according to claim 12, wherein the rotatable portion of the transmitter assembly (30) is further comprised of means to force engagement between the terminal ends of the series of paired conducting lines (11; 16; 17) of the sensor array and the individual electrodes (55) of the transmitter unit (30).

14. An assembly according to one or more of the preceding claims, wherein the transmitter unit (30) has circuitry to determine if the electrical connection has been made and an indicator (33) to confirm the electrical connection to a user.

15. An assembly according to one or more of the preceding claims, wherein a portion of the transmitter unit is transparent (32) and the terminal ends of the series of paired conducting lines (11; 16; 17) of the sensor array are visible through the transparent portion and in the aperture (31) of the transmitter unit (30).

16. **An assembly** to monitor incontinence in a patient comprising:
- a disposable garment (10) having an absorbent layer and formed from a fluid impermeable outer layer (20) having a sensor array (11; 16; 17) disposed on an inner surface thereof and in fluid communication with the absorbent layer and a non-woven comfort layer (21) covering the absorbent layer and disposed between the sensor array (11; 16; 17) and the absorbent layer and the patient,
- wherein the impermeable outer layer and the comfort layer (21) have a marginal edge having a terminal end, preferably with a flexibility and rigidity defined by the material layers forming the marginal end, and
- wherein the terminal end is intact across a transmitter-unit (30) connecting area consisting essentially of marginal edges of the fluid impermeable layer and the comfort layer (21), preferably in the absence of additional material adding flexibility or rigidity,
- the sensor array (11; 16; 17) comprised of a series of conducting lines (11; 16; 17) formed by conducting ink, parallel and of substantially equal width, layered on the inner surface of the external backing layer and in fluid communication with the absorbent material to form linear sensing regions for detecting a gradient of non-binary conductance values between the conducting lines (11; 16; 17), and
- wherein the conducting lines (11; 16; 17) are continuous and each terminate in the transmitter-unit (30) connecting area in a configuration matching a fixed array of electrodes in a reusable transfer unit, preferably in the absence of additional material adding flexibility or rigidity, and
- the reusable transmitter unit (30) sized and shaped to engage the transmitter-unit connecting area (17) and having an open position and a closed position, and
- wherein a housing of the transmitter unit (30) contains a fixed array of electrodes (55) and means to force the terminal ends of the series of conducting lines (11; 16; 17) at the intact linear segment of the marginal edge of the garment (10) into an electrical connection with the fixed array of electrodes (55),
- wherein the forcing means creates a multi-layered combination in the closed position comprising an individual electrode of the transmitter unit (30) in electrical connection with a single conducting line of the sensor array (11) at the intact linear segment, and a forcing means exerting to create the electrical connection.

17. An assembly according to claim 16, wherein the transmitter unit (30) has a groove traversing a lateral surface of the transmitter, wherein the groove has a depth to receive the marginal edge of the garment (10) and a width to receive the sensor array (11) in the transmitter-unit connecting area of the garment.

18. An assembly according to one or more of claim 16-17, wherein the transmitter unit (30) is further comprised of a series of projections aligned across the forcing means such that in the closed position the projections create the electrical connection by bringing a member of the series of paired conducting lines into electrical contact with a member of the fixed array of electrodes.

19. An assembly according to claim 18, wherein the projections penetrate the comfort layer (21) proximate to the member of fixed array of electrodes.

20. An assembly according to one or more of claim 16-19, wherein the terminal ends of the pairs of conducting lines are an array of parallel lines in the transmitter-unit connecting area spaced apart to match the distance between members of the fixed array of electrodes in the transmitter unit (30).

21. An assembly according to one or more of claim 16-20, wherein the terminal ends of the pairs of conducting lines (11) are bounded by a visual marker for aligning the terminal ends with an aperture in the housing of the transmitter unit (30) that receives the marginal edge of the garment (10).

22. An assembly according to one or more of claim 16-21, said assembly further comprising a computer network receiving wireless information from the transmitter unit (30) that identifies a single and unique electronic signature for each single garment (10).

23. **A network system** for detecting incontinent events in an individual patient comprising:
- a garment (10) having a sensor array (11) in fluid communication with absorbent material (20) disposed in the garment,
wherein the garment (10) has a marginal edge with a flexibility and rigidity defined by the material layers forming the marginal end layer, preferably in the absence of additional material adding flexibility or rigidity to an intact attachment region located at the marginal edge of the garment,
- a sensor array (11) having an electrical signature optionally unique to each garment and comprised of conducting lines (11) having terminal ends thereof aligned in the intact attachment region,
- a transmitter unit (30) having an aperture (31) sized and shaped to receive the intact attachment region, a fixed array of electrodes oriented to match the alignment of the terminal ends of the conducting lines (11),
- means for releasably securing the marginal edge to the transmitter unit (30),
- an indicator (33) confirming an electrical connection between the electrodes of the transmitter unit, and the terminal ends of the sensor lines and a connection to a wireless network;
- a data processing unit receiving data over the wireless network that correlates the presence of fluid in the absorbent material and detected by the sensor with the unique electronic signal of each garment (10), and
- a user interface to identify the individual patient with an incontinent event.

24. A network according to claim 23, wherein the method further comprises the step of:
the network system monitors a plurality of patients, and
the data processing unit distinguished between individual patients based on information received from the sensor array (11).
